(19) Europäisches Patentamt

European Patent Office

Office européen des brevets

(11) **EP 1 447 668 A2**

(12) **EUROPEAN PATENT APPLICATION**

(43) Date of publication:
**18.08.2004 Bulletin 2004/34**

(51) Int Cl.[7]: **G01N 33/68**, G06F 19/00

(21) Application number: **03257916.1**

(22) Date of filing: **16.12.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **20.12.2002 US 327261**

(71) Applicant: **Agilent Technologies, Inc.**
**Palo Alto, CA 94306 (US)**

(72) Inventors:
• **Thompson, Dean R.**
  **Fort Collins, Colorado 80526 (US)**
• **Apffel, James Alexander Jr.**
  **Mountain View, California 94040 (US)**

(74) Representative: **Tollett, Ian et al**
**Williams Powell**
**Morley House**
**26-30 Holborn Viaduct**
**London EC1A 2BP (GB)**

(54) **A method for improving data dependent ion selection in tandem mass spectroscopy of protein digests**

(57) Proteins in a sample are subjected to a computational digest to provide a set of predictive peptides for the protein. The predictive set of peptides are analyzed for their degree of prediction for the protein and rank-ordered to create a set of optimal predictor peptides. The optimal predictor peptides are then used to provide m/z ranges to the control software for the mass spectrometer to use as recommendations for ion selection for second stage analysis.

EP 1 447 668 A2

## Description

The present invention relates to a method for improving ion selection for second stage analysis in a tandem mass spectrometer. In particular, the invention relates to compounds, compositions and methods for determining the identity and sequence of proteins and peptides.

Protein sequencing has traditionally been carried out using Edman degradation. However, the technique is slow and can require significant amounts of the protein. In order to overcome the disadvantages of Edman degradation, several new techniques have been developed. The most common method makes use of mass spectroscopy.

There are currently two primary approaches to analysis of complex samples via mass spectroscopy. The first approach uses two-dimensional gel electrophoresis to separate intact proteins from the sample. The resulting protein spots on the gel are examined to determine a set of proteins of interest. The criteria used to determine proteins of interest is highly dependent on the biological question at hand, but often involves visual comparison of multiple gels. Each protein or peptide of interest is individually cut from the gel, digested via an appropriate proteolytic agent and the resulting peptide mixture analyzed using mass spectroscopy. The analyte may be introduced to the mass spectrometer via MALDI ionization (in which case all peptides from the protein are introduced simultaneously) or by electrospray ionization. With electrospray, the peptides are commonly separated using an online HPLC separation to simplify the peptide mixture being presented for mass analysis. In either case, the analyte consists of peptides from a relatively pure protein sample and current data-dependent mass selection techniques are usually adequate.

The second approach starts with the original protein sample. Typically, after reduction and alkylation, the entire sample is digested with an appropriate proteolytic agent, such as trypsin. The result is a hyper-complex peptide mixture containing all of the peptides from all of the proteins in the initial sample. This peptide mixture is then subjected to multiple dimensions of liquid phase separation, such as offline cation exchange HPLC followed by online reverse phase HPLC, with the final dimension being an online separation with the output being directly connected to an electrospray ionization source.

The ability to correctly identify the proteins in the original sample is dependent on obtaining adequate peptide coverage for the protein. While it is theoretically possible to identify a protein by correctly identifying a single peptide from the protein, typically between two and five peptides are required.

Peptides are typically identified via analysis of spectra obtained using tandem mass spectroscopy. In tandem mass spectroscopy, parent ions generated from a sample are fragmented to yield one or more daughter ions which are subsequently mass analyzed. Usually, parent ions are generated from a sample and passed through a first mass filter to select those ions having a particular mass-to-charge ratio. Typically, a narrow mass-to-charge window of about 2-4 Da, centered around the m/z ratio of the peptide to be analyzed, is selected. The selected ions are then fragmented to yield daughter ions that are then passed through to a second mass spectrometer and detected to produce a fragmentation or tandem spectrum. The chemical structures of unknown peptides are then determined using fragmentation spectra of the daughter ions.

The fragmentation spectra can be interpreted either manually, or by using computer-based methods, such as those based on graph theory and 'sub-sequencing' strategies or those that correlate the protein and peptide mass spectral data with sequence databases, thereby allowing for the rapid identification of proteins and peptides. Another approach for identifying peptide sequences is described in U.S. Patent No. 5,53 8,897 to Yates III *et al.* For each candidate sequence within the database spectrum, a theoretical fragmentation spectrum is formed according to a selected ion model of peptide fragmentation. The predicted theoretically derived mass spectra are compared to each of the experimentally derived fragmentation spectra by a cross-correlation function for scoring spectra.

With the hyper-complex peptide mixtures resulting from preseparation digestion of proteins, the ability to identify a specific protein is limited by the ability of the real-time algorithm used to select masses from the initial scan for fragmentation to correctly select a sufficient number of peptides from that protein. This ability is compromised by several factors. First, depending on the specific elution gradient used in the final HPLC separation, any particular peptide will have a finite period of time when it is present in the analyte subjected to the mass spectrometry. This period of time is frequently short in comparison to the duty cycle of the mass spectrometer, resulting in a limited number of opportunities for the peptide to be selected for MS/MS. Second, the real-time selection algorithm typically makes its selection based on relative ion intensity in the parent scan. Thus, given imperfect separation of peptides, the peptides from relatively abundant proteins tend to be selected over those from proteins with relatively low abundance. Third, since the selection algorithm has no knowledge of how the peptide masses that it selects map onto proteins (and cannot, due to real-time constraints) it will tend to select more peptides from a relatively abundant protein than are required to correctly identify that protein, wasting MS/MS timeslots that could be used to select peptides from less abundant proteins.

Most selection algorithms attempt to reduce the effects of this last behavior by using dynamic exclusion whereby a mass, once selected, will not be selected again for a fixed period of time, typically long enough for the elution of that peptide to be completed. However, with the hyper-complex mixtures resulting from preseparation digestion, there are often more peptides presented to the mass spectrometer during a particular period than can be selected. Most mass selection algorithms also have a provision for giving preferential treatment to a list of specific masses. This list is typically manually created, and therefore tends to be used to look for a small number of specific masses, to explicitly identify the presence of a particular compound. The result of these limitations is that current methods tend to miss peptides from proteins having lower abundances. There remains a need, therefore, for compositions and methods for selecting primary ions in tandem mass spectroscopic methods of assaying and sequencing proteins and peptides.

The present invention fulfills the above-described need. In one embodiment, the invention is directed to a method for improving ion selection for second stage analysis in a tandem mass spectrometer. The method comprises:

   identifying a set of proteins of interest from a set of proteins for which amino acid sequences are available;
   creating a set of optimal predictor peptides for the proteins of interest, wherein the optimal predictor peptides comprise a set of highly predictive peptides for each protein of interest;
   calculating a set of m/z ranges for each optimal predictor peptide that is observed if present in a sample being analyzed; and
   performing mass selection for second stage analysis by selecting ions found in the m/z ranges over ions having m/z values outside of the m/z ranges and having higher intensities.

In certain embodiments, the set of highly predictive peptides comprises about 5 peptides for each protein. In other embodiments, the set of most predictive peptides comprises about 3 peptides for each protein.
In still further embodiments, the set of highly predictive peptides is created by a method comprising subjecting the proteins of interest to a computational digest using the cleavage characteristics of a selected cleavage reagent to give a set of predicted peptides for the proteins;

   selecting a subset of the predicted peptides that provides a high degree of prediction;
   and rank-ordering the subset of predicted peptides to give the highly predictive peptides.

In certain embodiments, the cleavage reagent is selected from the group consisting of trypsin, chymotrypsin, protease, elastase, carboxypeptidase, papain, pepsin, proteinase K, thermolysin and subtilisin.
In other embodiments, the high degree of prediction is based on at least one factor selected from the group consisting of uniqueness of the peptide sequence, the charge state of a ion produced from the peptide sequence, the mass separation of the peptide from other peptides, the length of the peptide, the position of the peptide in the protein sequence, the presence of rare amino acids in the peptides and the presence of sequences capable of post-translational modification.
In yet further embodiments, the high degree of prediction is based on at least two factors selected from the group consisting of uniqueness of the peptide sequence, the charge state of a ion produced from the peptide sequence, the mass separation of the peptide from other peptides, the length of the peptide, the position of the peptide in the protein sequence, the presence of rare amino acids in the peptides and the presence of sequences capable of post-translational modification.
In another embodiment, the subject invention is directed to a method for selecting an ion for second stage analysis in a tandem mass spectrometer. The method comprises:

   creating a set of optimal predictor peptides for the proteins of interest, wherein the optimal predictor peptides comprise a set of most predictive peptides for each protein wherein the set of most predictive peptides is created by a method comprising subjecting the proteins in the sample to a computational digest using the cleavage characteristics of a selected cleavage reagent to give a set of predicted peptides for the proteins, selecting a subset of the predicted peptides that provides a high degree of prediction, and rank-ordering the subset of predicted peptides to give the most predictive peptides;
   calculating the m/z range for the peptides in the optimal predictor peptides; and
   selecting ions in the m/z range for second stage analysis.

In certain embodiments, the high degree of prediction is based on at least one factor selected from the group consisting of uniqueness of the peptide sequence, the charge state of a ion produced from the peptide sequence, the mass separation of the peptide from other peptides, the length of the peptide, the position of the peptide in the protein sequence, the presence of rare amino acids in the peptides and the presence of sequences capable of post-translational modification.
In further embodiments, the high degree of prediction is based on at least two factors selected from the group consisting of uniqueness of the peptide sequence, the charge state of a ion produced from the peptide sequence, the mass separation of the peptide from other peptides, the length of the peptide, the position of the peptide in the protein sequence, the presence of rare amino acids in the peptides and the presence of sequences capable of post-translational modification.
In certain embodiments, the set of most predictive peptides comprises about 2 to 5 peptides for each protein.
These and other aspects of the present invention will become evident upon reference to the following detailed description of a number of preferred embodiments.

Unless otherwise defined below, the terms used herein have their normally accepted scientific meanings. Definition of standard chemistry terms may be found in reference works, including Carey and Sundberg (1992) "Advanced Organic Chemistry 3rd Ed." Vols. A and B, Plenum Press, New York. The practice of the present invention will employ, unless otherwise indicated, conventional methods of mass spectroscopy, protein chemistry, biochemistry, recombinant DNA techniques and pharmacology, within the skill of the art. Such techniques are explained fully in the literature. See, for example, G. Barany and R.B. Merrifield (1980) "The Peptides: Analysis, Synthesis, Biology" Vol. 2, E. Gross and J. Meienhoffer, eds. Academic Press, New York., *Methods In Enzymology* (S. Colowick and N. Kaplan eds., Academic Press, Inc.); *Remington's Pharmaceutical Sciences,* 18th Edition (Easton, Pennsylvania: Mack Publishing Company, 1990); F.W. McLafferty (1993) "Interpretation of Mass Spectra" F.W. Benjamin, Inc, New York; D.C. Liebler (2002) "Introduction to Proteomics" Humana Press, New Jersey.

All publications, patents and patent applications cited herein, whether *supra* or *infra,* are hereby incorporated by reference in their entirety.

I. Definitions

It must be noted that, as used in this specification and the appended claims, the singular forms "a", "an" and "the" include plural referents unless the content clearly dictates otherwise. Thus, for example, reference to "an oligonucleotide" includes a mixture of two or more oligonucleotides, and the like.

A "protein" or a "polypeptide" is used in it broadest sense to refer to a compound of two or more subunit amino acids, amino acid analogs, or other peptidomimetics. The subunits may be linked by peptide bonds or by other bonds, for example ester, ether, etc. As used herein, the term "amino acid" refers to either natural and/or unnatural or synthetic amino acids, including glycine and both the D or L optical isomers, and amino acid analogs and peptidomimetics. A peptide of three or more amino acids is commonly called an oligopeptide if the peptide chain is short. If the peptide chain is long, the peptide is typically called a polypeptide or a protein. Full-length proteins, analogs, and fragments thereof are encompassed by the definition. The terms also include postexpression modifications of the polypeptide, for example, glycosylation, acetylation, phosphorylation, ubiquitination, and the like. Furthermore, as ionizable amino and carboxyl groups are present in the molecule, a particular polypeptide may be obtained as an acidic or basic salt, or in neutral form. A polypeptide may be obtained directly from the source organism, or may be recombinantly or synthetically produced. The following amino acid abbreviations are used throughout the text:

| | |
|---|---|
| Alanine: Ala (A) | Arginine: Arg (R) |
| Asparagine: Asn (N) | Aspartic acid: Asp (D) |
| Cysteine: Cys (C) | Glutamine: Gln (Q) |
| Glutamic acid: Glu (E) | Glycine: Gly (G) |
| Histidine: His (H) | Isoleucine: Ile (I) |
| Leucine: Leu (L) | Lysine: Lys (K) |
| Methionine: Met (M) | Phenylalanine: Phe (F) |
| Proline: Pro (P) | Serine: Ser (S) |
| Threonine: Thr (T) | Tryptophan: Trp (W) |
| Tyrosine: Tyr (Y) | Valine: Val (V) |

By "isolated" is meant, when referring to a polypeptide, that the indicated molecule is separate and discrete from the whole organism with which the molecule is found in nature or is present in the substantial absence of other biological macromolecules of the same type. The term "isolated" with respect to a polynucleotide is a nucleic acid molecule devoid, in whole or part, of sequences normally associated with it in nature; or a sequence, as it exists in nature, but having heterologous sequences in association therewith; or a molecule disassociated from the chromosome.

As used herein, a "biological sample" refers to a sample of tissue or fluid isolated from a subject. Typical samples include but not limited to, blood, plasma, serum, fecal matter, urine, bone marrow, bile, spinal fluid, lymph fluid, samples of the skin, secretions of the skin, respiratory, intestinal, and genitourinary tracts, tears, saliva, milk, blood cells, organs, biopsies and also samples of *in vitro* cell culture constituents including but not limited to conditioned media resulting from the growth of cells and tissues in culture medium, e.g., recombinant cells, and cell components.

As used herein, the terms "label" and "detectable label" refer to a molecule capable of detection, including, but not limited to, radioactive isotopes, fluorescers, chemiluminescers, chromophores, enzymes, enzyme substrates, enzyme cofactors, enzyme inhibitors, chromophores, dyes, metal ions, metal sols, ligands (e. g., biotin, avidin, strepavidin or haptens) and the like. The term "fluorescer" refers to a substance or a portion thereof which is capable of exhibiting fluorescence in the detectable range.

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

In one aspect, the invention identifies a set of potential peptides which are possibly present in the sample to be analyzed. This set of potential peptides is arrived at by a computational analysis of the actual or predicted amino acid sequence for a set of proteins of interest derived from a protein sequence database. The potential peptides resulting from each protein of interest are then rank-ordered according to the degree to which they are highly predictive of their source protein and the degree to which they may be reliably identified in real-time in the parent scans of the tandem mass spectrometer. A relatively small number of the potential peptides for each protein of interest is then selected on the basis of this rank-ordering to create a set of optimal predictor peptides. For each peptide in this set, a set of m/z ranges is calculated, based on the likely charge-states of the peptide and the resolution and mass accuracy of the mass spectrometer to be used for analysis. The resulting set of m/z ranges is then used to bias the selection of ions for a second stage analysis. The invention thus provides for improved coverage of proteins in a tandem mass spectroscopy analysis by better informing the real time ion selection algorithm.

Proteins of Interest

The proteins for use in the present invention can be any protein, such as, for example proteins associated with diseases, proteins that function to determine the phenotype of a cell, proteins indicative of cell cycle or activation state of a cell, proteins associated with a cell type, tissue or organ, and the like. The proteins thus include secreted proteins; integral membrane proteins, including receptors, cell adhesion molecules, and the like; cytoplasmic proteins; proteins from complexes, including ribosomal proteins, polymerase proteins, intracellular signal proteins, and the like; organelle proteins, including mitochondrial proteins, lysosomal proteins, nuclear proteins, endoplasmic reticulum proteins, and the like; and nucleic acid binding proteins, including histones, repressors, transcriptional activators, trans-acting enhancer factors, ribonuclear proteins, and the like.

Once a set of proteins of interest has been identified, the amino acid sequence for each protein in the set can be determined. In one aspect, the protein sequence can be obtained from the literature, such as for example, protein sequence databases. In addition, the sequences of the proteins can be obtained from a nucleotide database by converting the 3-base codons to a protein sequence. Thus, a number of sequence libraries can be used, including, for example, the Genpept database, the GenBank database, EMBL data library, the Protein Sequence Database, SWISS-PROT, and PIR-International, for example.

Protein Digestion

The protein or a set of proteins whose sequence is known or determined as described above can then be subjected to a computational digest. Typically, the identity of the protein digestion reagent is provided, or dependent on the type of experiment to be performed. The protein sequence together with the characteristics of the digestion reagent can then be used to create a set of predicted peptides for each protein. The proteins may be subjected to digestion with any of the well-known protein digestion reagents. Such reagents may be chemical or enzymatic. The range of protein cleavage reagents include digestion by proteases including papain, clostropain, trypsin, LysC, GluC and by chemical digestion, such as, for example, acid digestion, and cyanogen bromide.

Proteolytic enzymes such as endopeptidases, cleave proteins at known cleavage sites. Such enzymes are particularly useful for generating peptide fragments in a computational digest in accordance with the present invention. Proteases useful in practicing the present invention include trypsin, chymotrypsin, protease, elastase, carboxypeptidase, papain, pepsin, proteinase K, thermolysin and subtilisin (all of which can be obtained from Sigma Chemical Co., St. Louis, Mo.). The protease for use in practicing the present invention is selected such that the protease is capable of digesting the particular protein of interest. Papain cleaves on the carboxy-terminal side of Arg-X, Lys-X, His-X and Phe-X, and is a relatively mild protease that is commercially available in a highly purified form (Sigma). Clostropain cleaves on the carboxy-terminal side of arginine residues, and is preferably used if the preferred cleavage site is Arg-Tyr. Trypsin is the most commonly used reagent for protein digestion, with the enzyme cleaving the protein on the carboxy-terminal side of arginine and lysine residues. However, if larger fragments are preferred, LysC can be used to digest the protein. LysC only cleaves at lysine residues, therefore, on average produces larger fragments than trypsin.

In the computational digest method, an algorithm searches the protein sequence for the sequence residues that can serve as the cleavage site for the chosen digestion reagent. The first cleavage site and the second cleavage site can thus be identified. The sequences in between the cleavage sites are identified and stored as a separate sequence by the software program. For convenience, the sequence can be identified as peptide(n), where n serves as the identifier. The peptides thus obtained may range in size from 1 amino acid to 50 or more consecutive amino acids, preferably about 5 consecutive amino acids to about 20 consecutive amino acids, depending on the protein sequence, the digestion reagent, and the type of mass spectrometer to be used for analysis. Thus, the molecular weight for such peptides is from about 50 to 20,000 daltons.

Generating Optimal Predictor Peptides

In order to generate a set of mass to charge (m/z) ranges for use in ion selection for the second stage of the analysis, a set of peptides is identified that is most predictive for each protein. The set of peptides is referred to as the "Optimal Predictor Peptides."

In one aspect of the invention, the peptides from the computational digest are analyzed and rank-ordered such that they provide a high degree of prediction for the associated protein. In rank ordering the peptides, a number of factors can be considered, such as the uniqueness of the sequence, the charge states of the ions from the peptides, the degree of mass separation, the length of the peptide, the position of the peptide in the protein sequence, the presence of rare amino acids in the peptide sequence, the presence of post-translational modifications, and the like. One or more of these factors can be employed to rank-order the peptides for their degree of predictability. The most predictive peptides for each protein are then selected to create the "Optimal Predictor Peptides" set for the protein.

One of the factors that can be considered in rank-ordering the peptides is the uniqueness of the sequence. The sequence of the peptide can be analyzed to identify amino acids having identical mass, such as leucine and isoleucine, or having similar mass, such as glutamine and lysine, for example. The presence of such amino acid residues within the sequence of a pair of peptides will result in the peptides having similar mass in mass spectroscopic analysis. Thus, even though the peptides have sequences that are not identical, they will be considered identical from mass spectroscopy. Therefore, peptides having such amino acid residues have reduced predictability for the protein. The methods of the invention thus sums the number of occurrences of such amino acid residues in each peptide. The peptides with the lowest number are rank-ordered higher in their ability to predict the protein.

Another factor that can be considered in rank-ordering the peptides is the mass separation of the peptides. In this aspect of the invention, the mass of the peptides for each protein is calculated. In general, the mass of the peptides can be calculated by summing the masses of linear amino acid sequences. The mass calculated for each of the peptides is compared with the mass calculated for the other peptides. Peptides having m/z values that are separated from the m/z values of the other peptides can be more predictable. The peptides having greater mass separation can have a higher probability of being a pure selection thereby yielding a less complex spectrum in the second stage. These peptides are therefore assigned a higher predictability score in rank- ordering the peptides.

Another factor that can be considered in rank-ordering the peptides is the sequence length of the peptides. In this aspect of the invention, for each peptide identified in the computational digest, the number of amino acid residues can be summed to provide the length of the peptide. The length of the peptide may affect the ability of the peptide to be predictive of the source protein. Thus, very short peptides that are 1, 2, 3, 4, 5, or so amino acids in length, may not be very predictive of the protein. Similarly, very large peptides, such as 1000 amino acids or longer, may not be very predictive since they can, in some instances, be difficult to ionize. Therefore, peptides that are either very short or very long are assigned a lower rank-order.

Yet another factor that can be considered in rank-ordering the peptides is the position of the peptides in the protein sequence. In this aspect of the invention, the peptides that occur near the C-terminus of the protein may be more predictive of the protein. In addition, if the peptide is from a region of the protein entailing a high degree of homology, the peptide may not be very predictive of the protein. The degree of homology between the peptide and the protein can be calculated by the use of the blast algorithm (Altschutz *et al.* (1990) J. Mol. Biol. 215:403-410) or other techniques known to one skilled in the art. Such peptides are therefore assigned a lower rank-order. "Homology" refers to the percent similarity between two polynucleotide or two polypeptide moieties. Two or more polypeptide sequences are "substantially homologous" to each other when the sequences exhibit at least about 50% , preferably at least about 75%, more preferably at least about 80%-85%, preferably at least about 90%, and most preferably at least about 95%-98% sequence similarity over a defined length of the molecules. As used herein, substantially homologous also refers to sequences showing complete identity to the specified DNA or polypeptide sequence.

Another factor that can be considered in rank-ordering the peptides is the presence of rare amino acids within the sequence of the peptides. In this aspect of the invention, the peptide sequence is scanned for the presence of the rare amino acids. Peptides having the rare amino acids are assigned a higher rank-order.

Another factor that can be considered in rank-ordering the peptides, is the presence of sequences related to post-translations modifications of the peptides. Over 250 post-translational modifications have been described, including alkylation, (Saragoni *et al.* (2000) Neurochem. Res. 25:59-70), phosphorylation (Vanmechelen *et al.* (2000) Neurosci. Lett. 285:49-52), sulfation (Manzella *et al.* (1995) J Biol. Chem. 270:21665), oxidation or reduction (Magsino *et al.* (2000), Metabolism 49:799-803), ADP-ribosylation Galluzzo *et al.* (1995) Eur. J Immunol. 25:2932-9), hydroxylation (Brinckmann *et al.* (1999) J. Invest. Dermatol. 113:617), glycosylation (Johnson *et al.* (1999) Br. J. Cancer 81: 1188-95), glucosylphosphatidylinositide addition (Poncet *et al.* (1996) Acta Neuropathol. 91:400), ubiquitination (Chu *et al.* (2000) Mod. Pathol 13:420), translocation leading to a disease state (Reddy *et al.* (1999) Trends Neurosci. 22:248), and the like. For example, intracellular tyrosines are often subject to phosphorylation. In this aspect of the invention, the peptides having sequence that can be predicted to be subject to post-translational modifications are identified. Such peptides result in unpredictable mass modifications, and can therefore reduce the effectiveness of the peptide as a predictor. Peptides thus identified can be assigned a lower rank-order.

The peptides thus rank-ordered can then be used to generate the optimal predictor peptides. In one aspect, one of ordinary skill in the art identifies the relevant factors depending on the objectives of the experiment. Thus, for example, if the purpose of the experiment is to identify proteins having modifications at particular phosphorylation sites, then peptides containing amino acids that are susceptible to phosphorylation can be given greater preference over other criteria, such as for example the presence of unique amino acids. Once the most relevant criteria have been selected, the peptides from the computational digest are analyzed as described above. The peptides exhibiting the highest score in the relevant criteria are then grouped together thereby forming the optimal predictor peptides. Thus, a set of optimal predictor peptides is created by selecting the n most predictive peptides from each protein of interest. The value of n is chosen to be a small value that is consistent with accurate results from the database search algorithm to be used to interpret the resulting spectra.

In another aspect of the invention, a database of the optimal predictor peptides can be created for a particular type of experiment. For example, a particular set of optimal predictor peptides can be associated with proteins susceptible to phosphorylation. The database may be created by subjecting a range of samples to the methods described above and building up a database of optimal predictor peptides. The database can then be searched for optimal predictor peptides for that type of experiment.

Sequence and Identity Determination

The methods of the present invention are utilized to determine the sequence and/or identity of a protein. Various mass spectrometers may be used within the present invention. Representative examples include, triple quadrupole mass spectrometers, magnetic sector instruments (e.g., magnetic tandem mass spectrometer, JEOL, Peabody, MA); ion-spray mass spectrometers; electrospray mass spectrometers; laser desorption time-of-flight mass spectrometers; quadrupole ion-trap spectrometers; and a Fourier Transform Ion Cyclotron Resonance Mass Spectrometer (Extrel Corp., Pittsburgh, PA). In one aspect of the invention, an electrospray mass spectrometer (Agilent Technologies, Palo Alto, CA) is utilized to fragment the proteins, and a time-of-flight detector with better than 50 ppm mass accuracy is used to determine the sequence from the masses of the fragments.

Typically, the amino acid sequence of the proteins in a sample is obtained, either from the protein sequence databases or by predicting the amino acid sequences using the DNA sequence. For each protein sequence in the sample, a computational digest is performed using the cleavage characteristics of the cleavage reagent. The set of peptides thus obtained is predictive of the protein, and the set contains peptides that result from commonly missed cleavages. The predictive set of proteins is further analyzed and a subset of peptides providing high degree of prediction for the protein is selected. The peptides are rank-ordered to create a set of optimal predictor peptides as described above. For each peptide in the set of optimal predictor peptides, the m/z ranges and the likely charge states for the ion from the peptide are calculated from the predicted mass of the peptides. The resulting set of m/z ranges can then be provided to the control software for the mass spectrometer to be used as recommendations for ion selection for second stage analysis. The time-of-flight analysis of these daughters compared with the parent then allows identification of the constituents of the parent ion. Where a complete analysis of the sample is required, the experiments must be conducted for all masses present in the sample.

Below are examples of specific embodiments for carrying out the present invention. The examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

Example 1

The following is an example of implementing the invention.

Step 1: Identify a set of proteins of interest. In one embodiment of the invention, a list of the proteins in the proteome of the organism under study is generated.

Step 2: For each protein in the set, a list of possible peptides is created as created for that protein, given a particular proteolytic enzyme. For example, for a tryptic digestion, the sequence is broken at K-x and R-x where x is any amino acid except proline. For the purpose of creating this list assume that up to 2 cleavage sites could be missed, thus creating a list of peptides whose sequence overlaps to some degree, using standard techniques.

Step 3: Each peptide is scored according to the following criteria, and each score consists of a simple Boolean (True/False).

1. The theoretical mass of the peptide is unique relative to all other peptides (from all proteins) in the set, within a given tolerance (e.g. +/- 20ppm);
2. Sequence length of the peptide is >8 and <20 amino acids;
3. Peptide contains one of the following rare amino acids: cystine or tryptophan;

Step 4: A priority is assigned to each peptide by examining the set of scores for that peptide according to the following table:

| Score | Unique Mass | Good Length | Rare acid |
|-------|-------------|-------------|-----------|
| 1 | T | T | T |
| 2 | T | F | T |
| 3 | T | T | F |
| 4 | F | T | T |
| 5 | F | T | F |

Other combinations score 6

Step 5: The peptides for each protein are sorted in ascending score order. Within a score, peptides are sorted by decreasing sequence length.

Step 6. A list of mass ranges is assembled according to the following:

For each protein:

For peptides 1-3:

Calculate theoretical mass of the peptide by summing the residue masses of the amino acids in the sequence and adding the mass of a water molecule.

Calculate the tolerance at that mass according to the following formula:

$$(\text{Theoretical mass} / 1{,}000{,}000) * t$$

where t is the tolerance used in step 3 in parts per million.

Calculate the upper limit by adding the tolerance to the theoretical mass.

Calculate the lower limit by subtracting the tolerance from the theoretical mass.

Next peptide
Next protein

Accordingly, novel compounds and the use of the compounds for identifying, sequencing, and examining the differential expression of proteins and peptides have been disclosed. From the foregoing, it will be appreciated that, although specific embodiments of the invention have been described herein for purposes of illustration, various modifications may be made.

## Claims

1. A method for improving ion selection for second stage analysis in a tandem mass spectrometer, the method comprising:

identifying a set of proteins of interest from a set of proteins for which amino acid sequences are available;

creating a set of optimal predictor peptides for the proteins of interest, wherein the optimal predictor peptides comprise a set of highly predictive peptides for each protein of interest;

calculating a set of m/z ranges for each optimal predictor peptide that is observed if present in a sample being analyzed; and

performing mass selection for second stage analysis by selecting ions found in the m/z ranges over ions having m/z values outside of the m/z ranges and having higher intensities.

2. The method of claim 1, wherein the set of highly predictive peptides comprises about 5 peptides for each protein.

3. The method of claim 1, wherein the set of highly predictive peptides comprises about 3 peptides for each protein.

4. The method as claimed in any of claims 1 to 3, wherein the set of highly predictive peptides is created by a method comprising subjecting to proteins of interest to a computational digest using the cleavage characteristics of a selected cleavage reagent to give a set of predicted peptides for the proteins;

selecting a subset of the predicted peptides that provides a high degree of prediction;

and rank-ordering the subset of predicted peptides to give the most predictive peptides.

5. A method for selecting an ion for second stage analysis in a tandem mass spectrometer, the method comprising:

creating a set of optimal predictor peptides for the proteins of interest, wherein the optimal predictor peptides comprise a set of most predictive peptides for each protein wherein the set of most predictive peptides is created by a method comprising subjecting the proteins in the sample to a computational digest using the cleavage characteristics of a selected cleavage reagent to give a set of predicted peptides for the proteins, selecting a subset of the predicted peptides that provides a high degree of prediction, and rank-ordering the subset of predicted peptides to give the most predictive peptides;

calculating the m/z range for the peptides in the optimal predictor peptides; and

selecting ions in the m/z range for second stage analysis.

6. The method of claim 4 or 5, wherein the cleavage reagent is selected from the group consisting of trypsin, chymotrypsin, protease, elastase, carboxypeptidase, papain, pepsin, proteinase K, thermolysin and subtilisin.

7. The method as claimed in claim 4, 5 or 6 wherein the high degree of prediction is based on at least one factor selected from the group consisting of uniqueness of the peptide sequence, the charge state of a ion produced from the peptide sequence, the mass separation of the peptide from other peptides, the length of the peptide, the position of the peptide in the protein sequence, the presence of rare amino acids in the peptides and the presence of sequences capable of post-translational modification.

8. The method as claimed in claim 7, wherein the number of factors on which the high degree of prediction is based is at least two.

9. The method as claimed in any of claims 5 to 8, wherein the set of most predictive peptides comprises about 2 to 5 peptides for each protein.